# EUROPÄISCHE PATENTSCHRIFT

(11) **EP 1 139 099 B1**
(45) Veröffentlichungstag und Bekanntmachung des Hinweises auf die Patenterteilung: **23.09.2009**
(21) Anmeldenummer: 01103892.4
(22) Anmeldetag: 16.02.2001
(51) Int. Cl.: G01N 33/497, G01N 27/12

(54) **Betriebsverfahren für einen Alkoholsensor**
Operation method for an alcohol sensor
Procede d'utilisation pour un capteur d'alcool

(30) Priorität: 25.02.2000 DE 10008969
(43) Veröffentlichungstag der Anmeldung: 04.10.2001
(73) Patentinhaber: SIEMENS AKTIENGESELLSCHAFT, 80333 München (DE)
(72) Erfinder: Fleischer, Maximilian, Dr., 85635 Höhenkirchen (DE); Freitag, Reinhard, 81379 München (DE); Meixner, Hans, Prof., 85540 Haar (DE)

(56) Entgegenhaltungen:
- EP-A- 0 327 396
- DE-A- 4 443 142
- DE-C- 19 638 498
- FR-A- 2 642 172
- US-A- 3 780 311
- US-A- 3 903 726
- US-A- 4 274 425
- RETI F ET AL: "Detection of reducing gases in air by beta-Ga2O3 thin films using self-heated and externally (oven-) heated operation modes" SENSORS AND ACTUATORS B,CH,ELSEVIER SEQUOIA S.A., LAUSANNE, Bd. 34, Nr. 1-3, 1. August 1996 (1996-08-01), Seiten 378-382, XP004031745 ISSN: 0925-4005
- FLEISCHER M ET AL: "Selectivity in high-temperature operated semiconductor gas-sensors" SENSORS AND ACTUATORS B, ELSEVIER SEQUOIA S.A., LAUSANNE, CH, Bd. 52, Nr. 1-2, 15. September 1998 (1998-09-15), Seiten 179-187, XP004157831 ISSN: 0925-4005
- FLEISCHER M ET AL: "Fast gas sensors based on metal oxides which are stable at high temperatures" SENSORS AND ACTUATORS B,CH,ELSEVIER SEQUOIA S.A., LAUSANNE, Bd. 43, Nr. 1-3, 1. September 1997 (1997-09-01), Seiten 1-10, XP004103424 ISSN: 0925-4005

## Beschreibung

Die Erfindung betrifft ein Verfahren zum Betrieb eines Alkoholsensors unter Einsatz eines halbleitenden, beheizten, gassensitiven Materials. Das an diesem Gassensorelement abgegriffene elektrische Signal ist ein Maß für den Alkoholgehalt in einem Testgasvolumen.

Das Führen eines Kraftfahrzeuges ist in Deutschland derzeit nur zugelassen, wenn die Blutalkoholkonzentration des Fahrers maximal 0,5 Promille beträgt. Der gerichtlich anerkannte Alkoholisierungsgrad eines Kraftfahrers wird in Deutschland durch eine Blutentnahme mit anschließender Laboranalyse nachgewiesen. Dieser Maßnahme geht eine Atem-Alkoholmessung mit chemisch funktionierenden Teströhrchen voraus, die relativ ungenau ist. Eine andere Methode verwendet relativ teure Infrarotspektrometer.

Es gibt nach wie vor Bestrebungen, die Blutentnahme als einzige gerichtlich anerkannte Nachweismethode durch ein Messgerät zu ersetzen, bei dem die Messungen mit zwei von einander unabhängigen Messprinzipien durchgeführt werden. Mit Messungen anhand der Infrarotspektroskopie lassen sich relativ gute Messergebnisse erzielen. Allgemein werden bisher folgende Messprinzipien angewandt:
- Chemische Teströhrchen, z. B. von Firma Dräger. Hierbei ist ein definiertes Volumen vorgegeben (die Begrenzung geschieht durch das Fassungsvermögen eines aufblasbaren Kunststoffbeutels), wobei Atemluft einer Testperson durch ein in einem Röhrchen befindliches Granulat einer chemischen Substanz strömt. Die chemische Reaktion mit dem in der Atemluft enthaltenen Alkohol, in der Regel Äthylalkohol, führt zu einem von der Alkoholmenge abhängigen Farbumschlag des Granulates. Ein auf dem Röhrchen aufgedruckter Strich markiert die zulässige Grenze von derzeit 0,5 Promille. Diese Methode ist mit einer hohen Messungenauigkeit behaftet, so dass dadurch unnötige Blutentnahmen angeordnet werden, was mit sehr hohen Kosten und mit einem Eingriff in die persönliche Unversehrtheit verbunden ist. Hinsichtlich der vor einiger Zeit vorgenommenen Absenkung der Alkoholgrenze von 0,8 auf 0,5 Promille reicht diese Messmethode nicht aus bzw. es liegt eine sehr hohe Täuschungsgefahr vor.
- Die Infrarotspektroskopie, beispielsweise der "Alkomat" der Firma Siemens betrachtet die Intensitätsabschwächung durch Absorption von Infrarotstrahlung bei einer oder mehreren alkoholtypischen Wellenlängen. Dieses Verfahren ist sehr genau, aber auch sehr aufwendig. Für beweiskräftige Messungen, insbesondere im Zusammenhang mit polizeilichen Sanktionen existieren Standgeräte, die auf der Basis der Infrarotabsorption mit zwei separaten, auf unterschiedlichen Wellenlängen arbeitenden Detektoren arbeiten. Diese sind jedoch durch sehr hohe Kosten gekennzeichnet, und das Messprinzip eignet sich auf gar keinen Fall für tragbare Geräte.
- Die Alkoholmessung über elektrochemische Zellen (Brennstoffzellen) sieht wie folgt aus: Zwischen zwei Elektroden befindet sich ein Elektrolyt, der bei Zutritt von Sauerstoff und Alkoholdampf durch eine elektrochemische Reaktion ähnlich wie bei einer Batterie einen kleinen elektrischen Strom liefert. Nachteilig ist, dass die Reaktion temperaturabhängig ist und die Zellen nur eine begrenzte Lebensdauer aufweisen. Eine sehr langsame Messgeschwindigkeit verhindert darüber hinaus jegliche Differenzierung während einer Messung.
- Halbleitende Metalloxidsensoren wie beispielsweise Zinnoxidsensoren von der Firma Figaro: Nachteile liegen in der hohen Langzeitdrift sowie in hohen Querempfindlichkeiten, beispielsweise auf Feuchtigkeit. Für die kostengünstige Realisierung tragbarer Geräte sind beheizte Metalloxid-Halbleitergassensoren vorteilhaft. Auf dem Markt erhältlich sind Zinnoxidsensoren (SnO₂). Damit sind kostengünstige Sensoren erhältlich, die mit relativ einfacher Elektronik betrieben und ausgelesen werden können. Andererseits weisen diese Sensoren eine Reihe immanenter Nachteile auf. Die Zinnoxidsensoren besitzen untolerierbar hohe Querempfindlichkeiten auf andere ausgeatmete Alkohole, Ketone bzw. Aldehyde, was zu Fehlmessungen führt. Diese Sensoren benötigen eine sensorindividuelle Eichung der Gasempfindlichkeit, wodurch hohe Kalibrierkosten entstehen sowie häufige Nachkalibrierungen und insbesondere eine sehr lange Aufheizzeit. Aus diesem Grund sind diese Sensoren für tragbare Geräte, bei denen zur Reduzierung des Energieverbrauches die Sensorheizung erst unmittelbar vor der Messung betrieben werden soll, grundsätzlich nicht geeignet, wenn hochgenaue Messungen gefordert sind.
   Retí et. al. offenbaren in Sensors and Actuators B 34 (1996) 378-382 Galliumoxid sensoren, die Ethanol detektieren.

Aus der EP 0 327 396 A ist ein Alkoholsensor bekannt, der auf einem beheizten Zinnoxid-Gassensorelement beruht. Dabei wird ein zu untersuchender Volumenstrom von ausgeatmeter Luft in einen Haupt- und einen Nebenstrom unterteilt und das Gassensorelement ist im Nebenstrom angeordnet.

Ziel der Erfindung ist der Betrieb eines transportablen und hochgenau messenden Alkoholsensors.

Die Lösung dieser Aufgabe geschieht durch die Merkmalskombination von Anspruch 1.

Die Grundlagen eines Sensors, der in der Erfindung verwendet wird, sind eine gassensitive schicht aus Galliumoxid, insbesondere in der β-Phase, eine in der Regel elektrische Beheizung dieses Gassensorelementes, die Platzierung dieses Gassensorelementes in einem Gehäuse derart, dass der Volumenstrom eines Testgases in einem Nebenstrom am Gassensorelement vorbeistreicht, so dass dieses normalerweise nicht gekühlt wird, und das Abgreifen eines elektrischen Sensorsignals von der gassensitiven Galliumoxidschicht sowie dessen Auswertung.

Dabei wird das Sensorelement zur Alkoholmessung auf eine konstante Betriebstemperatur geheizt und ein Signal des Gassensorelementes zusätzlich zur Bestimmung des Volumenstromes des Testgases herangezogen, indem der Einfluss der Kühlwirkung der Testgasströmung auf das Signal des Gassensorelementes ausgewertet wird.

Galliumoxid in der β-Phase ist ein n-leitendes Halbleitermaterial mit freien Elektronen an seiner Oberfläche und der daraus resultierenden elektrischen Leitfähigkeit. In normaler Atmosphäre (Luft) wird Sauerstoff vom Sensor absorbiert und bindet damit die freien Elektronen des Sensors. Dadurch ist der Sensorwiderstand an Luft höher als im Vakuum. Umgekehrt reduziert sich der Widerstand des Sensorelementes, wenn ein reduzierendes Gas, wie z. B. Alkoholdampf, auf das Sensorelement trifft und sich mit dem vom Sensorelement gebundenen Sauerstoff verbindet, wobei wieder Elektronen freigesetzt werden.

Die Sensitivität, d. h. der Quotient aus dem Widerstand an Luft (Grundwiderstand) zu dem Widerstand in der zu messenden Gas-/Luftatmosphäre ist ein Maß für die Gaskonzentration. Mit dem Galliumoxidsensor können bei einigen Gassorten noch Konzentrationen von wenigen ppm nachgewiesen werden. Der Grundwiderstand des Sensors liegt an Luft betrieben, je nach Betriebstemperatur, im Bereich von ca. 10 kΩ (bei 900°C Betriebstemperatur) bis zu einigen MΩ (bei 650°C Betriebstemperatur) und sinkt beispielsweise bei einer Alkoholkonzentration von 0,5 mg/l um den Faktor 40. In diesem Fall würde beispielsweise eine Blutalkoholkonzentration von 1,0 Promille vorliegen.

Eine optimale Einstellung der Betriebstemperatur ist bei 900°C gegeben. Bei dieser Temperatur liegt eine nicht maximale, aber ausreichende Sensitivität des Gassensorelementes vor bei gleichzeitiger minimaler Querempfindlichkeit auf andere Gase, insbesondere auf Feuchtigkeit.

Es ist besonders vorteilhaft, die Größe des Gehäuses des Alkoholsensors und die Heizleistung des Gassensorelementes so aufeinander abzustimmen, dass von vorneherein eine Kondensation von Luft bzw. eine Betauungspunktunterschreitung im Gehäuse des Alkoholsensors nicht auftritt.

Das Ansprechverhalten des Gassensorelementes ist ausreichend schnell, um den zeitlichen Verlauf eines Testgasvolumenstromes auszudetektieren. Eine vorteilhafte Ausgestaltung der Erfindung sieht vor, das Gassensorelement selbst zur Bestimmung der Feuchtigkeit im Testgasvolumen einzusetzen. Dies beruht auf der Tatsache, dass der Sensor bei verschiedenen Temperaturen eine unterschiedliche Empfindlichkeit auf Wasser aufweist.

Bei der Verwendung des Gassensorelementes als Strömungssensors, beispielsweise zur Detektion des Testgasvolumenstroms, wird die Abhängigkeit des Heizungswiderstandes von der Temperatur ausgenutzt.

Im Folgenden werden anhand von schematischen Figuren Ausführungsbeispiele beschrieben:
- Figur 1: zeigt einen Alkoholsensor in seitlichem Schnitt,
- Figur 2: zeigt die Temperaturabhängigkeit des Heizwiderstandes von Ga₂O₃-Hochtemperatur-Gassensoren und
- Figur 3: zeigt die Kennlinie des Ga₂O₃-Alkoholsensors bei einer Betriebstemperatur von 900°C.

Mit der Erfindung kann beispielsweise ein Vortestgerät zur Alkoholkontrolle bei Personen realisiert werden, das genauer ist als die chemischen Teströhrchen nach dem Stand der Technik und gleichzeitig deutlich billiger ist als die Infrarotspektrometer.

Der Sensor weist wie die meisten Metalloxidsensoren eine gewisse Querempfindlichkeit zu brennbaren Gasen auf. Bei dem hier beschriebenen Alkoholsensor stellen jedoch die meisten Querempfindlichkeiten kein Problem dar, da in der Atemluft kaum störende Konzentrationen an brennbaren Gasen vorkommen. Restquerempfindlichkeiten in Bezug auf in der Atemluft eventuell vorkommende inhalierbare Lösungsmittel, wie es bei Stoffwechselkranken der Fall sein kann, sollen aufgrund ihrer Fahrtüchtigkeit einschränkenden Wirkung durch den Ga₂O₃-Sensor nachgewiesen werden können. An dieser Stelle wären zu nennen Stoffwechselprodukte wie Azeton (bei Diabetikern) und Azetaldehyd beispielsweise bei Tablettenmissbrauch. Ein Ansprechen auf derartige Stoffe soll unterdrückt werden. Dies ist bei einem Betrieb des Sensors bei Temperaturen oberhalb von 800°C, vorzugsweise 900°C gewährleistet. Zwar zeigt der Ga₂O₃-Sensor seine maximale Empfindlichkeit für Alkohol, gemeint ist Äthylalkohol, bei einer Betriebstemperatur von ca. 750°C. An dieser Stelle zeigt jedoch der Sensor noch zu viele Querempfindlichkeiten und reagiert zudem bei diesen Temperaturen für die geforderten Anwendungen als Alkoholtestgerät zu langsam. Bei einer Temperatur des Sensors von 900°C verliert der Sensor zwar etwas an Empfindlichkeit, dafür liegen die Ansprechzeiten jedoch im Bereich von nur einigen Zehntelsekunden bis zu wenigen Sekunden. Damit reagiert der Ga₂O₃-Sensor schneller als Brennstoffzellen auf Alkohol.

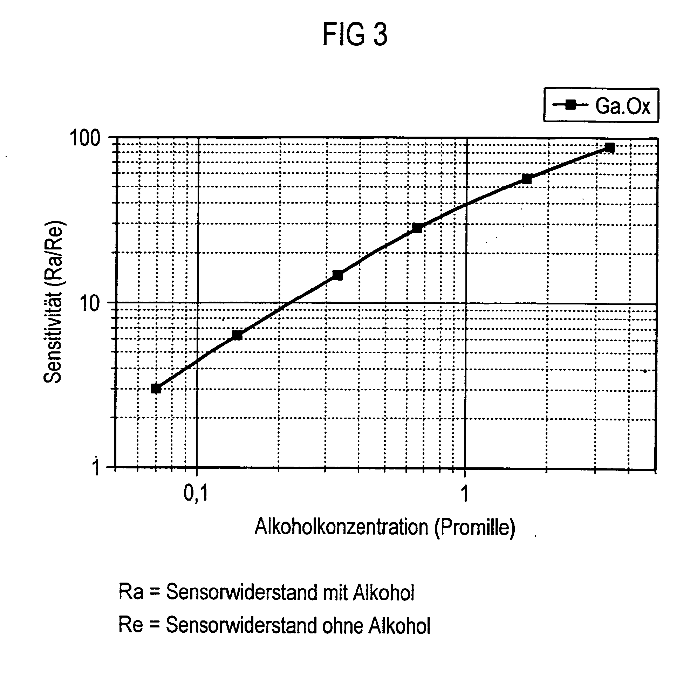

Die Kennlinie des bei einer Temperatur von 900°C betriebenen Ga₂O₃-Sensors zeigt Figur 3. Die Sensitivität stellt sich dar als der Quotient aus Sensorwiderstand mit Alkohol (Ra) und dem Sensorwiderstand ohne Alkohol (Re).

Für eine sehr präzise Messung der Alkoholkonzentration wirkt sich die verbleibende Restquerempfindlichkeit auf Wasser bzw. Wasserdampf störend aus. Eine Verfälschung der Messung des Luftwertes durch einen Feuchteanteil wirkt sich damit auf die Sensitivität und damit auf die gemessene Alkoholkonzentration aus. Daher ist die genaue Kenntnis der Luftfeuchtigkeit für die exakte Messung der Alkoholkonzentration notwendig. Die Messung der Luftfeuchte kann entweder durch eine separaten Feuchtigkeitssensor oder entsprechend der Erfindung mit dem Gassensorelement selbst durchgeführt werden. Dies beruht auf der Tatsache, dass der Sensor bei verschiedenen Temperaturen eine unterschiedliche Empfindlichkeit auf Wasser aufweist. Wird nun der Widerstand des Gassensorelementes an Luft, also ohne Alkohol, nicht nur bei Betriebstemperatur, sondern bei einer zweiten höheren oder tieferen Temperatur gemessen, beispielsweise durch Anlegen eines kurzen Temperaturimpulses, so lässt sich in Verbindung mit einer entsprechenden Auswerteelektronik die Feuchtigkeit der Umgebungsluft ermitteln und damit die Wasser-Querempfindlichkeit des Sensors ausrechnen.

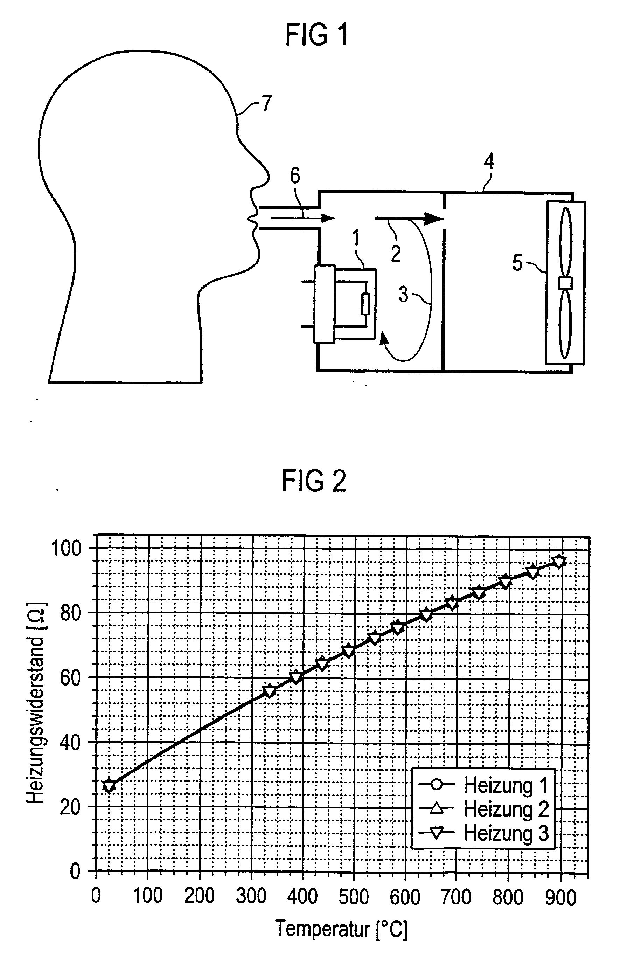

Das in Figur 1 dargestellte Beispiel eines Atemalkohol-Messrohres gewährleistet, dass der Sensor nicht durch die vorbeiströmende Atemluft gekühlt wird. Das Gassensorelement ist in dem Nebenstrom 3 eingebaut. Eine speziell entwickelte Heizungsregelung sorgt dafür, dass der Sensor sehr exakt auf der gleichen Temperatur gehalten wird, da der Grundwiderstand des Ga₂O₃-Sensors von seiner Betriebstemperatur abhängt und mit höherer Temperatur abfällt. Eine Abkühlung des Gassensorelementes würde somit bei der Messung des Alkoholgehaltes eines Testgasvolumens eine zu geringe Alkoholkonzentration vortäuschen. Mittels des Ventilators 5 ist eine Spülung der Kammern des Alkoholsensors möglich. Dies wird insbesondere zwischen zwei Alkoholmessungen vorgenommen werden. Die Kammer, in der sich der Sensor befindet, wird vorteilhafterweise so klein ausgelegt, dass die Strahlungswärme des Gassensors die Messkammer soweit aufheizt, dass eine Betauung der Messkammerwand sicher vermieden wird, da dies zu einer Verfälschung der Messergebnisse und auch zu längeren Wartezeiten bzw. Erholungszeiten zwischen zwei Messungen führen würde.

In Figur 1 ist dargestellt, wie eine Testperson 7 Atemluft, die das Testgas 6 darstellt, in einen Alkoholsensor bläst. Es entstehen ein Hauptstrom 2 und ein Nebenstrom 3. In Pfeilrichtung des Hauptstromes 2 betrachtet, kann das Testgas 6 über den bei einer Messung still stehenden Ventilator 5 ohne nennenswerten Widerstand nach außen strömen. Die Messung des Alkoholgehaltes im Testgas 6 erfolgt im Nebenstrom 3. Dies geschieht aus den oben genannten Gründen mit dem Ziel, eine konstante Betriebstemperatur des Gassensorelementes 1 beizubehalten. Für den Fall, dass das Sensorelement 1 neben der Messung des Alkoholgehalts im Testgas 6 auch zur Detektion des zeitlichen Verlaufes des Volumenstroms des Testgases 6 herangezogen wird, ist die Konstruktion des Gehäuses 4 des Alkoholsensors entsprechend auszulegen. Somit kann der Volumenstrom des Testgases 6 in Zusammenhang gebracht werden mit einer Signaländerung, die aufgrund einer Temperaturänderung am Sensorelement einhergeht. Unter Umständen kann eine vorhergehende Eichung notwendig sein.

Zur Sicherstellung einer verlässlichen Aussage bei einem Alkoholtest mit einem erfindungsgemäßen Alkoholsensor ist die Auswertung des zeitlichen Verlaufes beim Ausblasen des Testgases 6 zur Unterscheidung von Mund- und Lungenalkohol notwendig. Dabei sollte der von der Testperson 7 ausgeblasene Volumenstrom der Atemluft direkt und ohne Manipulationen in den Alkoholsensor gelangen. Wenn unmittelbar vor dem Test Alkohol konsumiert wurde, befindet sich in der Mundhöhle Alkohol, der zu Beginn des Ausatemvorgangs dem Testgerät zugeführt wird. Diese Zeitdauer ist sehr kurz, beispielsweise eine Sekunde. Danach wird das Testgerät mit originärer Ausatemluft aus der Lunge beaufschlagt. Um diese beiden Alkoholkonzentrationen zu unterscheiden, ist eine schnelle Sensorik mit Ansprechzeiten deutlich unter einer Sekunde nötig. Diese kurzen Ansprechzeiten lassen sich mit der innovativen Ga₂O₃-Sensorik realisieren, wenn Betriebstemperaturen des Sensorchips von ca. 900°C angewandt werden.

Zur sicheren Erkennung des Mundalkohols ist darüber hinaus die Zeiterfassung bzw. Aufzeichnung des Ausatemvorgangs erforderlich. Dies bedeutet, dass der Beginn des Ausatemvorgangs exakt erkannt werden soll. Es muss ein ausreichendes Ausatemvolumen pro Atemzug sichergestellt werden, um Täuschungsversuche zu erkennen. Hier ist beispielsweise ein "flaches Atmen" zu nennen, wobei versucht wird, dem Gerät ausschließlich Ausatemluft zuzuführen, die nicht in der Lunge war und dort vom Blut mit Alkohol gesättigt wurde. Wenn man von mechanischen Lösungen wie einem aufzublasenden Kunststoffbeutel in Verbindung mit einem chemischen Teströhrchen absieht, erfolgt die Aufzeichnung und Darstellung des Ausatemvorgangs durch eine geeignete Strömungssensorik. Eine Möglichkeit besteht darin, einen zusätzlichen Drucksensor in der Sensormesskammer einzubauen in Verbindung mit einem definierten Strömungswiderstand beim Gasauslass. Aus dem Zeitverlauf des Druckprofils kann dann auf die Ausatemmenge rückgeschlossen werden. Ein noch vorteilhafterer Weg, die benötigte Überwachung des Ausatemvorgangs zu realisieren, ist die direkte erfindungsgemässe Verwendung des Hochtemperatur-Ga₂O₃-Gassensors zusätzlich als Strömungssensor. Dabei wird eine weitere temperaturabhängige Funktion des Sensors ausgenützt. Nachdem der auf typischerweise 900°C beheizte Sensorchip durch Anströmung mit Ausatemluft definiert Wärme verliert, existiert eine Messgröße, die eine schnelle indirekte Messung der Strömung ermöglicht. Grundprinzip ist hierbei die Verwendung der Temperaturabhängigkeit des Heizwiderstandes wie sie in Figur 2 dargestellt ist. Das benötigte Signal zur Messung der Kühlung des Sensorchips lässt sich hier auf zwei verschiedene Weisen gewinnen.
a) Wenn die Heizung mit konstanter Spannung versorgt wird, erniedrigt sich der Widerstand der Heizung bei Abkühlung und es fließt ein höherer Strom, der als Sensorsignal zu detektieren ist. Die diesbezügliche Kennlinie ist in Figur 2 dargestellt. Die Bezeichnung Heater entspricht jeweils einer Heizschlange.
b) Wenn, wie bei vielen Anwendungsszenarien, von Hochtemperatur-Metalloxidsensoren die Sensortemperatur konstant gehalten wird, so wird dies über eine Regelung, die den Heizwiderstand auf konstantem Wert hält, getätigt. Bei einem Wärmeverlust durch Anblasen des Sensorelementes 1 wird also die Heizspannung hochgeregelt, wobei in diesem Fall sowohl eine Erhöhung von Heizstrom und Heizspannung bzw. dem Produkt aus beidem als Signal für die Strömung bzw. Strömungsmenge im Testgerät verwendet werden kann. Eine vorausgehende Eichung kann unter Umständen notwendig sein.

Mit dem beschriebenen Alkoholsensor ist demnach eine nicht invasive Messung des Alkoholgehalts (Äthylalkohol) im Blut lebender Menschen möglich. Die Lösung ist wiederverwendbar, störungssicher, transportabel und robust und kostengünstig. Absehbare Anwendungsgebiete sind
- Vortestgerät zur Alkoholkontrolle für die Polizei,
- Wegfahrsperre in Fahrzeugen, wobei der Fahrer in ein Messgerät ausatmen muss und das Fahrzeug nur gestartet werden kann, wenn der Alkoholgehalt eine bestimmte Schwelle nicht überschreitet - dies wird beispielsweise in den USA für Alkoholdelinquenten eingesetzt,
- Eine vergleichbare Anwendung wie im vorigen Punkt wäre denkbar für die Bedienung schwerer Maschinen und Baugeräte sowie für Flugzeuge,
- die angesprochenen Alkoholmessungen können beispielsweise freiwillig für Privatpersonen mit tragbaren, persönlichen Geräten erfolgen oder beispielsweise auch an Messstationen in Gastwirtschaften,
- derartige Alkoholsensoren können für sicherheitstechnische Anwendungen eingesetzt werden, beispielsweise bei dem Einlass von Massenveranstaltungen wie Fußballspielen oder Rockkonzerten.

### Bezugszeichenliste

- 1: Sensorelement
- 2: Hauptstrom
- 3: Nebenstrom
- 4: Gehäuse
- 5: Ventilator
- 6: Testgas
- 7: Testperson

## Patentansprüche

1. Verfahren zum Betrieb eines Alkoholsensors mit mindestens einem beheizten halbleitenden Gassensorelement (1) aus Galliumoxid und einem von einem Testgasvolumen durchströmten Gehäuse (4), wobei der Volumenstrom eines Testgases in einen Hauptstrom (2) und einen Nebenstrom (3) unterteilt ist und das mindestens eine Gassensorelement im Nebenstrom (3) positioniert ist, bei dem das Sensorelement (1) zur Alkoholmessung auf eine konstante Betriebstemperatur geheizt wird und ein Signal des Gassensorelementes (1) zusätzlich zur Bestimmung des Volumenstromes des Testgases herangezogen wird, indem der Einfluss der Kühlwirkung der Testgasströmung auf das Signal des Gassensorelementes (1) ausgewertet wird.

2. Verfahren nach Anspruch 1, bei dem die Betriebstemperatur ca. 900°C beträgt.

3. Verfahren nach Anspruch 1 oder 2, bei dem das Gehäuse (4) zwischen Alkoholmessungen mittels des Ventilators (5) gespült wird.

4. Verfahren nach einem der Ansprüche 1 bis 3, bei dem das Sensorelement (1) mit Luft ohne Alkohol zur Nullpunktbestimmung des Alkoholsensors gespült wird.

5. Verfahren nach Anspruch 4, bei dem bei zwei unterschiedlichen kurzzeitig eingestellten Temperaturen des Sensorelementes (1) der Feuchtigkeitsgehalt der Luft aus dem Verhältnis der beiden Sensorsignale ermittelt wird.

6. Verfahren nach einem der Ansprüche 1 bis 5, bei dem der Alkoholgehalt im Atemvolumen einer Testperson gemessen wird.

7. Verfahren nach Anspruch 6, bei dem der zeitliche Verlauf des Volumenstromes der Atemluft gemessen wird um zwischen Mund- und Lungenalkohol zu unterscheiden.

8. Verfahren nach Anspruch 7, bei dem ein minimales Atemvolumen je Atemzug überwacht wird.

## Claims

1. Method of operating an alcohol sensor with at least one heated semiconducting gas sensor element (1) made of gallium oxide and a housing (4) through which a test gas volume flows, the volumetric flow of a test gas being divided into a main flow (2) and a secondary flow (3) and the at least one gas sensor element being positioned in the secondary flow (3), in which method, for alcohol measurement, the sensor element (1) is set to a constant operating temperature and a signal of the gas sensor element (1) is additionally used for determining the volumetric flow of the test gas by evaluating the influence of the cooling effect of the test gas flow on the signal of the gas sensor element (1).

2. Method according to Claim 1, in which the operating temperature is about 900°C.

3. Method according to Claim 1 or 2, in which the housing (4) is purged by means of the fan (5) between alcohol measurements.

4. Method according to one of Claims 1 to 3, in which the sensor element (1) is purged with air without any alcohol to determine the zero point of the alcohol sensor.

5. Method according to Claim 4, in which the moisture content of the air is determined for two different temperatures of the sensor element (1), set for a short time, from the ratio of the two sensor signals.

6. Method according to one of Claims 1 to 5, in which the alcohol content in the tidal volume of respiratory air of a test person is measured.

7. Method according to Claim 6, in which the variation over time of the volumetric flow of the respiratory air is measured in order to differentiate between mouth alcohol and lung alcohol.

8. Method according to Claim 7, in which a minimal tidal volume of respiratory air is monitored for each breath.

## Revendications

1. Procédé pour faire fonctionner un capteur d'alcool, comprenant au moins un élément ( 1 ) de capteur de gaz à semiconducteur, chauffé, en oxyde de gallium et un boîtier ( 4 ) parcouru par un volume de gaz à tester, le courant en volume d'un gaz à tester étant subdivisé en un courant ( 2 ) principal et en un courant ( 3 ) secondaire et dans lequel on porte l'élément ( 1 ) de capteur pour la mesure de l'alcool à une température de fonctionnement constant et on tire parti d'un signal de l'élément ( 1 ) de capteur de gaz supplémentairement pour la détermination du courant en volume du gaz à tester, en exploitant l'influence de l'effet de refroidissement du courant du gaz à tester sur le signal de l'élément ( 1 ) de capteur de gaz.

2. Procédé suivant la revendication 1, dans lequel la température de fonctionnement est d'environ 900°C.

3. Procédé suivant la revendication 1 ou 2, dans lequel on balaye à l'aide du ventilateur ( 5 ) le boîtier ( 4 ) entre des mesures d'alcool.

4. Procédé suivant l'une des revendications 1 à 3, dans lequel on balaye l'élément ( 1 ) de capteur par de l'air sans alcool pour la détermination du point zéro du capteur d'alcool.

5. Procédé suivant la revendication 4, dans lequel on détermine, pour deux températures différentes et établies brièvement de l'élément ( 1 ) de capteur, la teneur en humidité de l'air à partir du rapport des deux signaux de capteur.

6. Procédé suivant l'une des revendications 1 à 5, dans lequel on mesure la teneur en alcool dans le volume respiratoire d'une personne à tester.

7. Procédé suivant la revendication 6, dans lequel on mesure la courbe en fonction du temps du courant en volume de l'air respiratoire pour distinguer entre l'alcool de bouche et l'alcool de poumon.

8. Procédé suivant la revendication 7, dans lequel on contrôle un volume minimum respiratoire à chaque aspiration.
